**Europäisches Patentamt**

**European Patent Office**

**Office européen des brevets**

(19)

(11) Veröffentlichungsnummer: **0 243 341 B1**

# (12) EUROPÄISCHE PATENTSCHRIFT

(45) Veröffentlichungstag der Patentschrift: **19.11.92**

(51) Int. Cl.⁵: **A61M 5/14**, A61B 10/00

(21) Anmeldenummer: **87890076.0**

(22) Anmeldetag: **15.04.87**

(54) **Biopsieeinrichtung zur Gewinnung von Gewebeproben und Applikation von Substanzen in einem Arbeitsgang.**

(30) Priorität: **21.04.86 AT 1041/86**
**13.04.87 AT 914/87**

(43) Veröffentlichungstag der Anmeldung:
**28.10.87 Patentblatt 87/44**

(45) Bekanntmachung des Hinweises auf die
Patenterteilung:
**19.11.92 Patentblatt 92/47**

(84) Benannte Vertragsstaaten:
**BE CH DE ES FR GB GR IT LI LU NL SE**

(56) Entgegenhaltungen:
**EP-A- 0 107 810**
**DE-A- 3 522 782**
**FR-A- 2 326 941**

(73) Patentinhaber: **IMMUNO Aktiengesellschaft**
**Industriestrasse 67**
**A-1221 Wien(AT)**

(72) Erfinder: **Zatloukal, Kurt, Dr.**
**Johann Loserthgasse 2**
**A-8010 Graz(AT)**
Erfinder: **Dinges, Hans Peter, Dr.**
**Jaritzweg 43**
**A-8045 Graz(AT)**

(74) Vertreter: **Wolfram, Gustav, Dipl.-Ing.**
**Patentanwälte Sonn, Pawloy, Weinzinger &**
**Wolfram, Riemergasse 14**
**A-1010 Wien(AT)**

**Beschreibung**

Die Erfindung betrifft eine Biopsieeinrichtung zur Gewinnung von Gewebsproben mit einer zwei- oder mehrlumigen Biopsiekanüle, die einen Biopsiekanal mit über seine gesamte Länge konstantem Querschnitt sowie mindestens einen Applikationskanal aufweist und die an ihrem proximalen Ende mit Anschlußmöglichkeiten für eine Saug- sowie mindestens eine Applikationsvorrichtung und an ihrem vorderen Ende mit einer Schneide versehen ist, wobei mindestens ein Applikationskanal durch ein exzentrisch über die Biopsiekanalwand geschobenes Rohr gebildet ist.

Es ist bekannt, daß zur Entnahme von Gewebsproben aus tierischen und menschlichen Organen verschiedene bioptische Techniken auf dem Prinzip einer Einkanalnadel mit Saugvorrichtung und/oder Schneidvorrichtung, mit und ohne Trokar existieren. Bekannt sind weiters mehrlumige Kanülen und Nadeln (DE-C - 818 246 und DE-A - 26 43 594), die für eine Biopsieentnahme und Substanzapplikation in einem Arbeitsgang allerdings nicht geeignet sind.

Zur Saugbiopsie ist zu bemerken: Die bekannteste Saugbiopsietechnik basiert auf dem von Menghini angegebenen Prinzip (Menghini, G. 1957: Un effettivo progresso nella tecnica della puntura biopsia del fegato. Rass. Fisiopat. clin. Ter. 29, 756). Dabei handelt es sich um eine im Durchschnitt 1,4 mm im Durchmesser haltende Hohlnadel mit einer Ansatzmöglichkeit für eine Spritze, mit der nach Durchstechen der Haut und vor der eigentlichen Organpunktion ein negativer Druck (Sog) angelegt wird. Die Organpunktion (Leber) erfolgt dann bei anhaltendem Sog im Bruchteil einer Sekunde.

Zur Schneidbiopsie ist zu bemerken: Die Schneidbiopsie erfolgt im Prinzip mit einer am vorderen Ende zugeschliffenen Kanüle. Schneidvorrichtung und Lumen der Kanüle sind während des Punktionsvorganges durch ein in das Kanülenlumen eingelegtes Stilett geschützt, das nach der Punktion entfernt wird. Erst dann wird aus dem punktierten Organ unter Sogwirkung bei drehender, vorwärts gerichteter Bewegung ein zylindrisches Gewebsstück herausgeschnitten (z.B.: Tru-Cut Nadel, Fa. Travenol).

Bei allen derzeit üblichen bioptischen Techniken muß mit Komplikationen gerechnet werden, die von der Art der Punktion (wie dem zu punktierenden Organ und der angewandten Technik) und vom Allgemeinzustand des Patienten abhängen. Die Hauptkomplikationen bei der Saugbiopsie der Leber stellen in erster Linie starke Blutungen, gallige Bauchfellentzündungen und der Pneumothorax dar (Lindner, H. 1967: Grenzen und Gefahren der perkutanen Leberbiopsie mit der Menghini-Nadel.

Dtsch. med. Wschr. 39, 1751; Piccinino, F., Sagnelli E., Pasquale, G., Guisti G. 1986: Complications following percutaneous liver biopsy. J. Hepatology 2, 165). Schneidbiopsien vom Lungengewebe weisen eine relativ hohe Komplikationsrate durch Blutungsereignisse und Pneumothorax auf (McEvoy, R.D., Begley, M.D., Antic, R. 1983: Percutaneous Biopsy of Intrapulmonary Mass Lesions. Cancer 51, 2321). Auch für Nierenbiopsien und Biopsien anderer Organe gilt in erster Linie eine schwere Blutung als bedeutendste Komplikation.

Um diesen Komplikationen zu begegnen, wurde die anschließende Plombierung des Stichkanales mit resorbierbarem Material empfohlen, um insbesondere Blutungskomplikationen hintanzuhalten (Riley, S.A., Irving, H.C., Axon, A.T.R., Ellis, W.R., Lintott, D.J., Losowsky M.S. 1984: Percutaneous liver biopsy with plugging the needle track: a safe method for use in patients with impaired coagulation. Lancet, Aug. 25, 1984, 436). Derartige Techniken setzen aber eine lange Verweildauer der Punktionsnadel im Organ voraus, was insbesondere bei Leberpunktionen eine neuerliche Ursache für Komplikationen darstellt (Thaler, H. 1982: Leberbiopsie, Springer-Verlag, Heidelberg-New York).

Aus dem prioritätsälteren nachveröffentlichten Dokument DE-A - 35 22 782 ist eine doppellumige Punktionskanüle zur Follikelpunktion im Rahmen der extrakorporalen Befruchtung mit einem größerlumigen Saugkanal zum Absaugen des Follikelinhalts und einem kleinerlumigen Spülkanal zum Zuführen von Spülflüssigkeit in das Follikelinnere beschrieben. Die Punktionskanüle ist als doppelwandiges Rohr ausgebildet, welches aus zwei koaxial und fest ineinander gepaßten Rohren besteht. Die Punktionskanüle weist eine angeschärfte Einstichspitze mit Hohlschliff auf, wobei die Schneide vom Außenrohr gebildet ist.

Die Erfindung stellt sich die Aufgabe, eine Biopsieeinrichtung der eingangs beschriebenen Art zu schaffen, mit der sowohl die Gewinnung von Gewebsproben als auch die Applikation von Substanzen in einem Arbeitsgang in kürzester Zeit (unter 1 s) möglich ist.

Diese Aufgabe wird erfindungsgemäß dadurch gelöst, daß die Schneide alleine von einem spitzwinkelig ausgebildeten Ende der Biopsiekanalwand gebildet ist und die Biopsiekanalwand mit ihrer Schneide aus dem Rohr vorragt und das Rohrende mit der Biopsiekanalwand einen stumpfen Winkel einschließt.

Vorzugsweise endet die Biopsiekanalwand rechtwinkelig zur Längsachse des Biopsiekanales.

Vorteilhaft ist die Biopsiekanalwand im Querschnitt teilweise abgeflacht, so daß zwischen der abgeflachten Seite der Biopsiekanalwand und dem Rohr ein Applikationskanal gebildet ist und zwischen der nicht abgeflachten Seite der Biopsiekanalwand und dem Rohr ein Flächenkontakt besteht.

Eine bevorzugte Ausführungsform ist dadurch gekennzeichnet, daß an der Nadel in an sich bekannter Weise ein zweiteiliger Kontaktmechanismus befestigt ist, wobei dessen proximaler Teil mit der Kanüle in fester Verbindung steht und der distale bewegliche Teil durch eine Feder in Ausgangsstellung gehalten wird, so daß nach Erreichen einer vorbestimmten Einstichtiefe dieser Teil durch die Körperoberfläche so verschoben wird, daß ein oder mehrere Kontakte zwischen dem proximalen starren und dem distalen beweglichen Teil geschlossen werden, die die Steuerung eines Applikationssystems abhängig von der Einstichtiefe und Punktionsgeschwindigkeit ermöglichen, und weiters durch Erreichen des Anschlages des distalen beweglichen Teiles an den proximalen Teil die Punktionstiefe begrenzt wird.

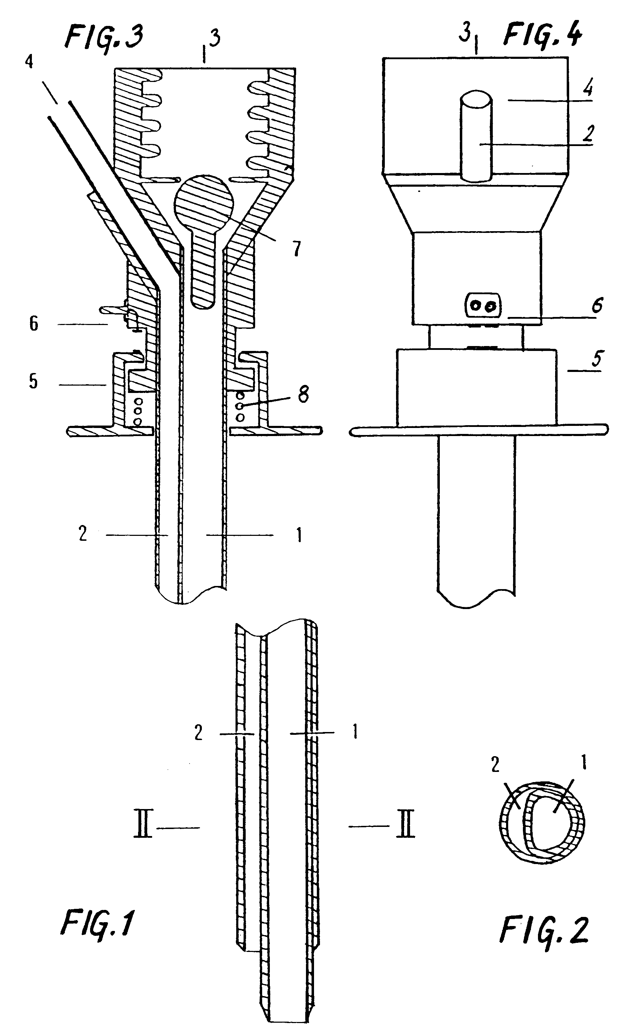

Ein Ausführungsbeispiel der Biospieeinrichtung ist in der Zeichnung dargestellt. Fig. 1 stellt einen Längsschnitt und Fig. 2 einen Querschnitt nach der Linie II-II der Fig. 1 des schneidenden Endes einer Zweikanalnadel dar. Es zeigen weiters die Fig. 3 einen Längsschnitt durch einen Kontaktmechanismus und Fig. 4 eine Ansicht desselben für die in den Fig. 1 und 2 dargestellte Zweikanalnadel.

Die Punktionsnadel weist einen Biopsiekanal 1, der den größten Nadelquerdurchmesser miterfaßt, auf. Wie aus Fig. 1 hervorgeht, erfolgt die Trennung gegenüber dem Applikationskanal 2 derart, daß im Biopsiekanal 1 keine Ecken und Kanten entstehen, die eine Qualitätsbeeinträchtigung der Gewebsproben bewirken könnten. Als Applikationskanal fungiert ein vom Hauptkanal abgetrennter kleinerer Raum der Punktionsnadel. Die Trennung der Kanäle ist durch zwei aneinander angepaßte, exzentrisch ineinander geschobene dünnwandige Rohre bewirkt.

Das schneidende Ende ist vom rechtwinkelig zur Längsachse endenden Biopsiekanal 1 alleine gebildet, dessen Wand spitzwinkelig geschliffen ist. Der durch ein exzentrisch über die Biopsienadelwand geschobenes Rohr entstandene Applikationskanal 2 reicht nicht bis zum schneidenden Ende. Seine Wand ist am Ende stumpfwinkelig geschliffen, so daß ein fließender Übergang der Außenwand des Biopsiekanales 1 in die Außenwand des Applikationskanales 2 in dem Abschnitt, in dem sich beide Kanäle berühren, bewirkt wird.

Am Ansatzende der Nadel (Fig. 3, 4) befindet sich ein das Lumen der Nadel nicht vollständig verschließender Bolzen 7, der ein Absaugen der Gewebsprobe in das bei 3 anschließbare Unterdrucksystem (z.B. Spritze mit Arretierung) verhindert. 4 stellt die Ansatzmöglichkeit für ein Applikationssystem dar, dessen Anordnung und Gestaltung (z.B. Konus, Gewinde etc.) an die jeweiligen Erfordernisse (z.B. unter Druck stehendes System) angepaßt werden kann. Die Begrenzung der Punktionstiefe und die Steuerung des Applikationssystems erfolgt durch einen der Nadel aufsitzenden Mechanismus, bestehend aus einem an der Kanüle befestigten und einen oder mehrere Kontaktpunkte aufweisenden Teil 6 und einen oder mehrere Kontaktpunkte aufweisenden beweglichen Teil 5, der durch eine Feder 8 in Ausgangsstellung gehalten wird. Nach Erreichen einer bestimmten Punktionstiefe wird Teil 5 nach Berührung mit der Körperoberfläche in Richtung Teil 6 bewegt, bis der Anschlag erreicht ist und der Kontakt geschlossen wird. Die Zahl der Anordnung von Kontaktpunkten kann den Erfordernissen zur Steuerung des Applikationssystems angepaßt werden. Anstelle des skizzierten elektrischen Steuerungssystems ist auch ein mechanisches System vorstellbar, ohne daß dadurch das Wesen der Erfindung eine Änderung erfährt.

Die Punktion erfolgt nach der Menghini-Technik, bei der nach Durchstechen der Haut ein Unterdruck mit Hilfe einer an der Stelle 3 angesetzten, arretierbaren Spritze erzeugt wird. Anschließend wird die Nadel weiter in das zu punktierende Gewebe (Organ) eingestochen. Nach Erreichen des Anschlages 5 wird die Nadel sofort zurückgezogen. Die Injektion von Substanzen (z.B. Blutgerinnungssubstanzen, vasoaktive Pharmaka, Zytostatika, Antibiotika etc.) wird automatisch durch den Auslösemechanismus nach Erreichen einer vorbestimmbaren Einstichtiefe ausgelöst.

Die Konstruktion ermöglicht eine Gewebsentnahme und eine mit dem Punktionsvorgang koordinierte Applikation von Substanzen in einem Arbeitsgang. Dadurch wird im Vergleich zu bisher bekannten Methoden die Eingriffsdauer stark verkürzt. Die Anordnung der Kanäle gewährleistet trotz des Vorhandenseins zusätzlicher Kanäle einen minimalen Kanülengesamtdurchmesser bei gleichbleibender Qualität der für die histologische Befundung entnommenen Gewebsprobe. Diese Nadel ermöglicht u.a. die unkomplizierte Plombierung des Punktionskanales mit gerinnungsaktiven Substanzen. Die Indikationen für Punktionen können dadurch wesentlich erweitert werden, was eine verbesserte Diagnostik und Therapie bedeutet.

**Patentansprüche**

1. Biopsieeinrichtung mit einer zwei- oder mehrlumigen Biopsiekanüle, die einen Biopsiekanal (1) mit über seine gesamte Länge konstantem Querschnitt sowie mindestens einen Applikationskanal (2) aufweist und die an ihrem proximalen Ende mit Anschlußmöglichkeiten für eine Saug- sowie mindestens eine Applikationsvorrichtung und an ihrem vorderen Ende mit einer Schneide versehen ist, wobei mindestens ein Applikationskanal durch ein exzentrisch über die Biopsiekanalwand geschobenes Rohr gebildet ist, dadurch gekennzeichnet, daß die Schneide alleine von einem spitzwinkelig ausgebildeten Ende der Biopsiekanalwand gebildet ist und die Biopsiekanalwand mit ihrer Schneide aus dem Rohr vorragt und das Rohrende mit der Biopspiekanalwand einen stumpfen Winkel einschließt.

2. Biopsieeinrichtung nach Anspruch 1, dadurch gekennzeichnet, daß die Biopsiekanalwand rechtwinkelig zur Längsachse des Biopsiekanales (1) endet.

3. Biopsieeinrichtung nach Anspruch 1 oder 2, dadurch gekennzeichnet, daß die Biopsiekanalwand im Querschnitt teilweise abgeflacht ist, so daß zwischen der abgeflachten Seite der Biopsiekanalwand und dem Rohr ein Applikationskanal (2) gebildet ist und zwischen der nicht abgeflachten Seite der Biopsiekanalwand und dem Rohr ein Flächenkontakt besteht.

4. Biopsieeinrichtung nach einem der Ansprüche 1 bis 3, dadurch gekennzeichnet, daß an der Nadel in an sich bekannter Weise ein zweiteiliger Kontaktmechanismus befestigt ist, wobei dessen proximaler Teil (6) mit der Kanüle in fester Verbindung steht und der distale bewegliche Teil (5) durch eine Feder (8) in Ausgangsstellung gehalten wird, so daß nach Erreichen einer vorbestimmten Einstichtiefe dieser Teil (5) durch die Körperoberfläche so verschoben wird, daß ein oder mehrere Kontakte zwischen dem proximalen starren (6) und dem distalen beweglichen Teil (5) geschlossen werden, die die Steuerung eines Applikationssystems abhängig von der Einstichtiefe und Punktionsgeschwindigkeit ermöglichen, und weiters durch Erreichen des Anschlages des distalen beweglichen Teiles (5) an den proximalen Teil (6) die Punktionstiefe begrenzt wird.

## Claims

1. A biopsy device comprising a two- or multilumen biopsy cannula including a biopsy channel (1) having a constant cross section over its total length as well as at least one application channel (2) and which, on its proximal end, is provided with connection facilities to an aspiration means as well as to at least one application means and, on its front end, is provided with a cutting edge, wherein at least one application channel is formed by a tube eccentrically slipped over the wall of the biopsy channel, characterized in that the cutting edge alone is formed by an acute-angularly designed end of the biopsy channel and the wall of the biopsy channel projects out of the tube by its cutting edge and the tube end encloses an obtuse angle with the wall of the biopsy channel.

2. A biopsy device according to claim 1, characterized in that the wall of the biopsy channel terminates at a right angle relative to the longitudinal axis of the biopsy channel (1).

3. A biopsy device according to claim 1 or 2, characterized in that the wall of the biopsy channel is partially flattened in terms of cross section such that an application channel (2) is formed between the flattended side of the wall of the biopsy channel and the tube and a surface contact prevails between the non-flattened side of the wall of the biopsy channel and the tube.

4. A biopsy device according to any one of claims 1 to 3, characterized in that a two-part contacting mechanism is fastened to the needle in a manner known per se, its proximal part (6) being firmly connected with the cannula and the distal, movable part (5) being maintained in the starting position by a spring (8) such that this part (5), after having reached a predetermined puncture depth, is displaced through the surface of the body in a manner that one or several contacts between the proximal, firm part (6) and the distal, movable part (5) are closed, thus enabling the control of an application system as a function of the puncture depth and the puncture speed, and, furthermore, the puncture depth is limited by the distal, movable part (6) striking against the proximal part (6).

## Revendications

1. Dispositif de biopsie comportant une canule de biopsie à deux ou plusieurs lumières présentant un canal de biopsie (1) de section constante sur toute sa longueur ainsi qu'au moins un canal d'administration (2) et munie à son extrémité proximale de possibilités de raccord d'un dispositif d'aspiration et d'au moins un dispositif d'administration et à son extrémité frontale d'un tranchant, au moins un canal d'administration étant formé par un tube enfilé excentriquement sur la paroi du canal de biopsie, caractérisé en ce que le tranchant est formé uniquement parune extrémité façonnée à angle aigu de la paroi du canal de biopsie et que la paroi du canal de biopsie dépasse par son tranchant du tube et que l'extrémité du tube forme un angle obtus avec la paroi du canal de biopsie.

2. Dispositif de biopsie selon la revendication 1, caractérisé en ce que la paroi du canal de biopsie se termine à angle droit par rapport à l'axe longitudinal du canal de biopsie (1).

3. Dispositif de biopsie selon la revendication 1 ou 2, caractérisé en ce que la paroi du canal de biopsie est partiellement aplatie dans sa section transversale de sorte qu'est formé un canal d'administration (2) entre le côté aplati de la paroi du canal de biopsie et le tube et qu'il se crée un contact superficiel entre le côté non aplati de la paroi du canal de biopsie et le tube.

4. Dispositif de biopsie selon une des revendications 1 à 3, caractérisé en ce qu'un mécanisme de contact en deux parties est fixé de façon connue en soi à l'aiguille, sa partie proximale (6) étant en liaison fixe avec la canule et la partie mobile distale (5) étant maintenue dans sa position initiale par un ressort (8), de sorte qu'après l'atteinte d'une profondeur d'enfoncement déterminée à l'avance cette partie (5) est déplacée par la surface corporelle de telle sorte qu'un ou plusieurs contacts s'établissent entre la partie rigide proximale (6) et la partie distale mobile (5), permettant la commande d'un système d'administration indépendamment de la profondeur d'enfoncement et de la vitesse de la ponction et qu'en outre la profondeur de la ponction est limitée par la venue en butée de la partie distale mobile (5) sur la partie proximale (6).

*FIG.3*

*FIG.4*

*FIG.1*

*FIG.2*